(19) Europäisches Patentamt
European Patent Office
Office européen des brevets

(11) Veröffentlichungsnummer : **0 150 474**
**B1**

# (12) EUROPÄISCHE PATENTSCHRIFT

(45) Veröffentlichungstag der Patentschrift : **17.08.88**

(51) Int. Cl.[4] : **C 07 D471/04** // (C07D471/04, 209:00, 221:00)

(21) Anmeldenummer : **84116106.0**

(22) Anmeldetag : **21.12.84**

(54) **Verfahren zur Herstellung von 5,6-Dihydro-pyrrolo[2,1-a]isochinolinen.**

(30) Priorität : **13.01.84 DE 3401018**

(43) Veröffentlichungstag der Anmeldung : **07.08.85 Patentblatt 85/32**

(45) Bekanntmachung des Hinweises auf die Patenterteilung : **17.08.88 Patentblatt 88/33**

(84) Benannte Vertragsstaaten : **AT BE CH DE FR GB IT LI LU NL SE**

(56) Entgegenhaltungen :
**GB-A- 1 153 670**
**JOURNAL OF MEDICINAL CHEMISTRY, Band 11, Juli 1968, Easton, Pennsylvania C. CASAGRANDE "Synthesis and Pharmacological Eveluation of Some Pyrrolo (2,1-a)isoquinolines" Seiten 765-770**
**THE JOURNAL OF ORGANIC CHEMISTRY, Band 40, Nr. 6, 1975, Easton, Pennsylvania F.M. HERSHENSON "Synthesis of Ring-Fused Pyrroles" Seiten 740-743**
**JOURNAL OF THE CHEMICAL SOCIETY, Perkin I, 1976, London R.M. ACHESON "Addition Reactions of Heterocyclic Compounds" Seiten 1908-1911**

(73) Patentinhaber : **BOEHRINGER INGELHEIM KG**
**Postfach 200**
**D-6507 Ingelheim am Rhein (DE)**
**BE CH DE FR IT LI LU NL SE AT**
**Boehringer Ingelheim International G.m.b.H**

**D-6507 Ingelheim am Rhein (DE)**
**GB**

(72) Erfinder : **Lösel, Walter, Dr.**
**Im Herzenacker 26**
**D-6535 Gau-Algesheim (DE)**
Erfinder : **Roos, Otto, Dr.**
**Elsheimer Strasse 36**
**D-6501 Schwabenheim (DE)**
Erfinder : **Schnorrenberg, Gerd, Dr.**
**Fichtenweg 13**
**D-6507 Ingelheim am Rhein (DE)**

**EP 0 150 474 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Verschiedene Derivate des 5,6-Dihydropyrrolo[2,1-a]isochinolins besitzen eine Reihe wertvoller pharmakologischer Eigenschaften (C. Casagrande, A. Invernizzi, R. Ferrini und G.G. Ferrari, J. Med. Chem. 11, 765, 1968 ; C. Casagrande, A. Invernizzi, R. Ferrini und G. Miracoli, Farmaco, Ed. Sci. 27, (12), 1029-44 (1972) ; Siphar, S.A. (Erf. G. Ferrari und C. Casagrande), Brit. Pat. 1, 153, 670 (29.05.1969) [Chem. Abstr. 71, 81215g] ; N. Nakayama, N. Imamura, T. Kanazawa und S. Yoneda, Heterocycles 20, (1), 168, (1983)). Trotz ihrer pharmakologischen Bedeutung wurde diese Verbindungsklasse wegen unzureichender Synthesemethoden bisher nur wenig bearbeitet.

Bei der Mehrzahl der literaturbekannten Synthesen wird der Tricyclus durch Umsetzung tautomeriefähiger 1-Alkyl-3,4-dihydroisochinoline mit 1,2-Biselektrophilen geschlossen. (C. Casagrande, A. Invernizzi, R. Ferrini und G.G. Ferrari, J. Med. Chem. 11, 765, 1968 ; J. Thesing und H.F. Funk, Chem. Ber. 91, 1546-51 (1958) ; Y. Ban und M. Terashima, Chem. Pharm. Bull, 13 (7) 775-85 (1965) ; S. Sakai, A. Kubo, M. Inaba, M. Katagiri und K. Tanna, Yakugaku Zasshi 86 (9), 856-8 (1966) [Chem. Abstr. 65, 18558a (1966)] ; H. Meyer, Liebigs Ann. Chem. 1981, 1534-1544). Wegen der begrenzten Auswahl an geeigneten Reaktionspartnern ist hier die Funktionalität der Pyrrolsubstituenten, meist Alkyl-, Aryl-, Esterfunktionen, nur in engen Grenzen variierbar. Andere Methoden finden nur in speziellen Fällen Anwendung (N. Nakayama, N. Imamura, T. Kanazawa und S. Yoneda, Heterocycles 20, (1), 168 (1983) ; Ph. Cauwel und J. Gardent, Tetrahedron Lett. 1972 (27) 2781-4 ; F.M. Hershenson, J. Org. Chem. 1975, 40 (6), 740-5 ; E. Breuer, S. Zbaida, J. Pesso und I. Ronen-Braunstein, Tetrahedron 1977, 33 (10), 1145-8 ; S. Kano, T. Yokumatsu, Y. Yuasa und S. Shibuya, Heterocycles, 19, (11),, 2143-5 (1982) ; W.E. McEwen, I.C. Wang Huang, C.P. Cartaya Marin, F. McCarty, E. Marmugi Segnini, Ch. M. Zepp III und J.J. Lubinkowski, J. Org. Chem. 1982, 47, 3098-3105) oder wegen ihrer Reaktionsmechanismen Interesse (M. Hanaoka, A. Wada, Y. Sakamoto, M. Ichihara, S. Yasuda und T. Imanishi, Heterocycles 16, (11), 1933-6 (1981) ; G. Blasko, G. Dörnvei, M. Barczai-Beke, P. Péchy und C. Szantay, Heterocycles 20, (2), 273-278 (1983)).

Die Erfindung schafft eine einfache und effektive Methode zur Darstellung neuer 5,6-Dihydro-pyrrolo-[2,1-a]isochinoline durch basenkatalysierte Umlagerung der leicht zugänglichen aus der DE-A 31 43 876 bekannten 1-(3-Furyl)-3,4-dihydroisochinoline.

Gegenstand der Erfindung ist ein Verfahren zur Herstellung von 5,6-Dihydro-pyrrolo[2,1-a]isochinolinen der allgemeinen Formel I

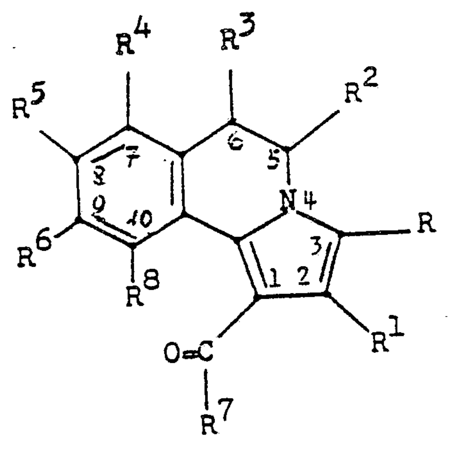

(I)

worin

R, $R^2$, $R^3$, $R^7$, die gleich oder verschieden sein können, Wasserstoff oder Alkyl mit 1 bis 4 C-Atomen,

$R^1$ Cyano, Hydroxycarbonyl, Alkoxycarbonyl mit 1 bis 4 C-Atomen, eine primäre Aminocarbonylgruppe oder eine sekundäre Aminocarbonylgruppe R'—NH—CO—, in der der Substituent R' eine gegebenenfalls substituierte aliphatische, cycloaliphatische, araliphatische, aromatische oder heterocyclische Gruppe ist,

$R^4$ und $R^8$, die gleich oder verschieden sein können, Wasserstoff, Hydroxy, Alkoxy, Alkylthio mit jeweils bis zu 4 C-Atomen oder eine $—NR^9R^{10}$-Gruppe,

$R^5$ und $R^6$, die gleich oder verschieden sein können, Hydroxy, Alkoxy oder Alkylthio mit jeweils 1 bis 4 C-Atomen oder gemeinsam eine $—O—CH_2—O—$, $—O—CH_2—CH_2—O—$ oder —O—CH=CH—O-Gruppe,

$R^9$ Wasserstoff oder Alkyl mit 1 bis 4 C-Atomen,

$R^{10}$ gegebenenfalls durch Hydroxy, Methoxy oder Furfuryl substituiertes Alkyl mit 1 bis 4 C-Atomen bedeuten.

Das Verfahren ist dadurch gekennzeichnet, daß man ein 1-(3-Furyl)-3,4-dihydro-isochinolin der allgemeinen Formel II

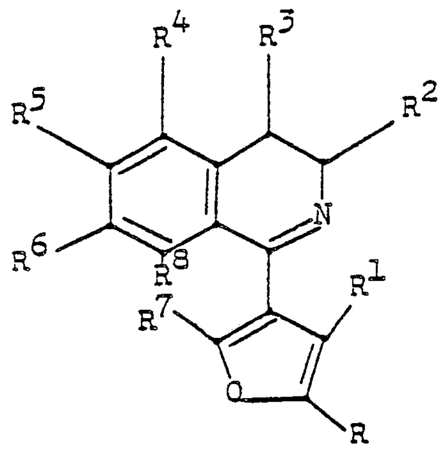

(II)

worin R sowie $R^1$ bis $R^8$ die oben angegebenen Bedeutungen besitzen, in einem primären oder sekundären Amin auf mindestens 80 °C mehrere Stunden erhitzt, das Amin entfernt, das Reaktionsprodukt mit verdünnter Säure behandelt und das Reaktionsprodukt reinigt und isoliert.

Als Amine können primäre und sekundäre aliphatische, cycloaliphatische, araliphatische Amine eingesetzt werden. Bevorzugt sind Morpholin, Piperidin, 2-(3,4-Dimethoxyphenyl)-ethylamin, 1-Amino-2-propanol oder Methoxy-ethylamin.

Das Reaktionsprodukt wird bei der Aufarbeitung normalerweise mit Säuren, vorzugsweise verdünnten Mineralsäuren behandelt, um etwa entstehende Azomethine zu spalten und die letzten Spuren des Amins aus dem Reaktionsprodukt zu beseitigen.

Die Reaktionsdauer ist von der Umsetzungstemperatur abhängig. Bei Temperaturen um 100 °C sind Reaktionszeiten bis zu 16 Stunden erforderlich. Bei Temperaturen von 200 °C genügt eine Reaktionsdauer von 1-2 Stunden. Bei Verwendung höher siedender primärer oder sekundärer Amine reicht es aus, die Ausgangsverbindung in dem Amin als Lösungsmittel unter Rückfluß zu erhitzen. Bei niedriger siedenden Aminen kann die Reaktion auch im geschlossenen System z. B. einem Autoklav oder im Bombenrohr durchgeführt werden.

Nach Aufarbeitung des Reaktionsansatzes erhält man die Endverbindungen in 70-98 %iger Ausbeute, die nach Elementaranalyse und Massenspektrum zu den Ausgangsverbindungen der allgemeinen Formel II isomer sind. Die Endverbindungen der allgemeinen Formel I fallen normalerweise als NMR-spektroskopisch reine Produkte an.

Unter den gewählten Umlagerungsbedingungen bleiben auch bei Verwendung primärer Amine Esterfunktionen normalerweise erhalten und es findet keine Umwandlung in entsprechende Amide statt.

Die Umlagerung der 1-(3-Furyl)-3,4-dihydro-isochinoline der allgemeinen Formel II in die 5,6-Dihydro-pyrrolo[2,1-a]-isochinoline der allgemeinen Formel I macht sich in den $^1H$-NMR-Spektren vor allem im Verschwinden der Protonensignale des Furansystems bemerkbar. Für die freien Basen der Ausgangsverbindungen der allgemeinen Formel II liegen die beiden Dubletts (J = 1,5 Hz) zwischen $\delta = 7{,}58$ und $\delta = 8{,}01$ ppm, für die entsprechenden Hydrochloride im Bereich $\delta = 8{,}20\text{-}8{,}68$ ppm.

An ihrer Stelle erscheinen in den Umlagerungsprodukten der allgemeinen Formel I neue Protonenresonanzen bei $\delta = 6{,}70\text{-}6{,}87$ ppm und $\delta = 9{,}09\text{-}10{,}67$ ppm, die nach Form und Lage dem Proton [3-H] im Pyrrolring und einem Aldehydproton bei Verbindungen der allgemeinen Formel I, worin $R^7$ Wasserstoff bedeutet, zuzuordnen sind. Als weiterer Ausdruck der tiefgreifenden Strukturänderung beim Übergang der Verbindungen der allgemeinen Formel II in die Endprodukte der allgemeinen Formel I findet man eine ausgeprägte Tieffeldverschiebung des Aromatenprotons [10-H]. Diese Zuordnung ergibt sich aus der beträchtlichen Abhängigkeit des chemischen Verschiebungswertes dieses Signals von der Substitution am C-1, wodurch die räumlich nahe Plazierung beider Funktionen belegt wird.

Die Struktur der Endverbindung kann darüber hinaus unabhängig von den spektroskopischen Befunden anhand der Folgeprodukte abgesichert werden. Die 5,6-Dihydro-pyrrolo[2,1-a]isochinoline der allgemeinen Formel I weisen im Pyrrolring eine Keto- bzw. Aldehydcarbonylfunktion und eine Carbonsäure-carbonylfunktion auf, die sich in vielfältiger Weise abwandeln läßt.

Mit der erfindungsgemäßen Umwandlung der 1-(3-Furyl)-3,4-dihydro-isochinoline in die 5,6-Dihydro-pyrrolo[2,1-a]isochinoline der allgemeinen Formel I steht ein neuer präparativ einfacher Zugang zu dieser Substanzklasse zur Verfügung. Der Vorzug dieses Verfahrens besteht in der Möglichkeit, funktionelle Gruppen regioselektiv in den Pyrrolring einzuführen.

Die nach dem Verfahren der Erfindung herstellbaren neuen 5,6-Dihydro-pyrrolo[2,1-a]isochinoline der allgemeinen Formel I sind wertvolle Ausgangsverbindungen zur Herstellung von pharmazeutisch wertvollen Pyridazino[4',5':3,4]pyrrolo[2,1-a]3,4-11,12-tetrahydroisochinolinen. Die zuletzt genannte Verbindungsklasse weist muscarinagonistische, calciumantagonistische, antihypoxietotische, nootrope, herzwirksame, stoffwechselaktive und blutdrucksenkende Wirkungen auf.

Die folgenden Beispiele erläutern das erfindungsgemäße Verfahren.

Schmelzpunkte : Apparat nach Tottoli (Firma Büchi), nicht korrigiert. — Elementaranalysen : Mikroanalytisches Laboratorium der Fa. Boehringer Ingelheim KG — IR-Spektren : Perkin-Elmer 257 (in Nujol : Intensitätsangaben : s = stark, m = mittel, w = schwach, br = breit). — 1H-(90 MHz)-Spektren : Bruker WH 90, Tetramethylsilan als innerer Standard ; δ = Werte) — Massenspektren : Varian MAT-CH 7 (70 eV ; Direkteinlaßbedingungen ; Verdampfungstemperatur ca. 200 °C) — Dünnschichtchromatographie (DC) : DC-Fertigplatten Kieselgel $F_{254}$ (Fa. Merck) : Fließmittelsysteme Methylenchlorid/Methanol = 100 : 10 ;

Die als Ausgangsverbindungen der allgemeinen Formel II benötigten 3-[3,4-Dihydro-isochinolinyl-(1)]-furan-4-carbonsäurederivate können nach den Angaben der DE-A-31 43 876 hergestellt werden.

1-Formyl-5,6-dihydro-pyrrolo[2,1-a]isochinolin-2-carbonsäurederivate

Allgemeine Vorschrift

Je 50 mmol des 1-(3-Furyl)-3,4-dihydro-isochinolins der allgemeinen Formel II werden unter $N_2$-Schutz in 25 ml des angegebenen, frisch destilliertem Amins 1 bis 2 Stunden bei Siedetemperatur gerührt. Nach beendeter Umsetzung wird im Vakuum zur Trockne eingedampft, der Rückstand in Methylenchlorid gelöst, nacheinander mit verdünnter Salzsäure und Wasser gewaschen, getrocknet und eingedampft. Der Rückstand wird mit Essigester verrieben und abgesaugt. Die NMR-spektroskopisch einheitlichen Rohprodukte können ohne weitere Reinigung verarbeitet werden. Zur Analyse werden Proben der Umsetzungsprodukte nach säulenchromatographischer Reinigung (Kieselgel/Methylenchlorid/Methanol (100 : 2)) aus den angegebenen Lösungsmitteln kristallisiert.

Beispiel 1

1-Formyl-5,6-dihydro-8,9-dimethoxy-pyrrolo[2,1-a]isochinolin-2-carbonsäureethylester durch Umlagerung von 3-[3,4-Dihydro-6,7-dimethoxy-isochinolinyl-(1)]-furan-4-carbonsäure-ethylester mit Morpholin.

Ausbeute 98 % ; Schmelzpunkt 185-187 °C (aus Essigester)

IR : 3 110 w (CH), 3 060 w (CH), 1 730 sh, 1 700 s (CHO), 1 655 s (COOR) 1 600 m, 1 570 m $cm^{-1}$. $^1H$-NMR ($CD_3OD$) : δ = 1.37 (t, J = 7 Hz ; 3H, $CH_3$—$CH_2$), 3.00 (t, J = 7 Hz ; 2H, Ar—$CH_2$), 3.91, 4.01 jeweils (s, 3H, $OCH_3$), 4.07 (t, J = 7 Hz ; 2H, $CH_2$—$CH_2$—N), 6,71 (s, 1H, 3—H), 7.33 (s, 1H, 7—H), 8.62 (s, 1H, 10—H), 10.67 (s, 1H, CHO). MS : m/z = 329 (100 %, $M^+$), 300 (41 %, M— CHO), 283 (M— $C_2H_5OH$).

$C_{18}H_{19}NO_5$ (329,35)

| | | | |
|---|---|---|---|
| Ber. : | C 65.64 | H 5.81 | N 4.25 % ; |
| Gef. : | C 65.93 | H 6.01 | N 4.00 %. |

Beispiel 2

1-Formyl-5,6-dihydro-8-methoxy-9-hydroxy-pyrrolo[2,1-a]-isochinolin-2-carbonsäureethylester durch Umlagerung von 3-[3,4-Dihydro-6-methoxy-7-hydroxy-isochinolinyl-(1)]-furan-4-carbonsäureethylester mit Morpholin.

Ausbeute 68 % ; Schmelzpunkt 218-222 °C (aus Essigester)

IR : 3 400-3 100 m br (OH), 3 070 w, 1 700 s (CHO), 1 640 s (COOR), 1 600 s, 1 590 s, 1 530 s, $cm^{-1}$. $^1H$-NMR ($CDCl_3$) : δ = 1.36 (t, J = 7 Hz ; 3H, $CH_3$—$CH_2$), 2.98 (t, J = 7 Hz ; 2H, Ar—$CH_2$), 3.99 (s, 3H, $OCH_3$), 4.04 (t, J = 7 Hz ; 2H, $CH_2$—$CH_2$—N), 4.33 (q, J = 7 Hz ; 2H, $CH_3$—$CH_2$), 5.73 (s, breit, 1H, OH), 6.72 (s, 1H, 3—H), 7.34 (s, 1H, 7—H), 8.36 (s, 1H, 10—H), 10.66 (s, 1H, CHO).

$C_{17}H_{17}NO_5$ (315.33)

| | | | |
|---|---|---|---|
| Ber. : | C 64.75 | H 5.43 | N 4.44 % ; |
| Gef. : | C 64.94 | H 5.52 | N 4.19 %. |

Beispiel 3

1-Formyl-5,6-dihydro-8,9-dioxymethylen-pyrrolo[2,1-a]isochinolin-2-carbonsäureethylester durch Umlagerung von 3-[3,4-Dihydro-6,7-dioxymethylen-isochinolinyl-(1)]-furan-4-carbonsäureethylester-hydrochlorid mit 2-(3,4-Dimethoxyphenyl)-ethylamin.

Ausbeute 73 % ; Schmelzpunkt 167-168 °C (aus Essigester)

IR : 3 125 m, 1 690 s (CHO), 1 660 s (COOR), 1 620 w $cm^{-1}$. $^1H$-NMR ($CDCl_3$) : δ = 1.36 (t, J = 7 Hz ; 3H, $CH_3$—$CH_2$), 2.98 (t, J = 6,5 Hz ; 2H, Ar—$CH_2$), 4.05 (t, J = 6,5 Hz ; 2H, $CH_2$—$CH_2$—N), 4.32 (q, J = 7 Hz ; 2H, $CH_3$—$CH_2$), 5.98 (s, 2H, O—$CH_2$—O), 6.70 (s, 1H, 3—H), 7.34 (s, 1H, 7—H), 8.32 (s, 1H, 10—H), 10.66 (s, 1H, CHO).

$C_{17}H_{15}NO_5$ (313.31)

Ber. : C 65.17 H 4.83 N 4.47 % ;

Gef.: C 65.33 H 4.96 N 4.41 %.

Beispiel 4

1-Formyl-5,6-dihydro-8,9-dimethoxy-pyrrolo[2,1-a]isochinolin-2-carbonitril

a) durch Umlagerung von 3-[3,4-Dihydro-6,7-dimethoxy-isochinolinyl-(1)]-furan-4-carbonitril-hydrochlorid mit Morpholin.

Ausbeute 83 % ; Schmelzpunkt 221-222 °C (aus Essigester)

IR : 3 100 w (CH), 2 220 s (CN), 1 660 s (CHO), 1 600 m 1 575 m $cm^{-1}$. $^1$H-NMR ($CDCl_3$) : δ = 3.06 (t, J = 7 Hz ; 2H, Ar—$CH_2$), 3.91, 3.98 jeweils (s, 3H, $OCH_3$), 4.11 (t, J = 7 Hz ; 2H, $CH_2$—$CH_2$—N), 6.76 (s, 1H, 3—H), 7.20 (s, 1H, 7—H), 8.10 (s, 1H, 10—H), 10.14 (s, 1H, CHO). MS : m/z = 282 (100 %, $M^+$), 267 (19 %, M— $CH_3$), 239 (9 %, M— $CH_3$—CO).

$C_{16}H_{14}N_2O_3$ (282.30)

Ber.: C 68.07 H 5.00 N 9.92 % ;
Gef.: C 68.07 H 5.12 N 9.83 %.

b) durch Umlagerung in Dimethylformamid (DMF) 14 g (43.92 mmol) der Ausgangsverbindung der allgemeinen Formel II wurden in 200 ml reinem DMF 45 Minuten zum Sieden erhitzt. Danach wurde das Lösungsmittel im Vakuum abgezogen, der Rückstand zwischen Methylenchlorid und Wasser verteilt, über Natriumsulfat getrocknet, an Kieselgel (Eluent : Methylenchlorid/Methanol (100 : 5)) gereinigt und aus Essigester kristallisiert (hellorange Kristalle). Das Umsetzungsprodukt stimmt laut Schmelzpunkt 220-221 °C, IR- und NMR-Spektren, Elementaranalyse und chromatographischem Verhalten überein mit dem nach a) hergestelltem Endprodukt.

$C_{16}H_{14}N_2O_3$ (282.30)

Ber.: C 68.07 H 5.00 N 9.92 % ;
Gef.: C 68.19 H 5.12 N 9.92 %.

Beispiel 5

1-Formyl-5,6-dihydro-8,9-dimethoxy-pyrrolo[2,1-a]isochinolin-2-carbonsäure-(2-hydroxypropyl)-amid

a) durch Umlagerung von 3-[3,4-Dihydro-6,7-dimethoxy-isochinolinyl-(1)]-furan-4-carbonsäure-(2-hydroxy-propyl)-amid mit Morpholin.

Ausbeute 67 % ; Schmelzpunkt 196-197 °C (aus Ethanol/Ether)

IR : 3 260-3 140 m, 3 060 m, 1 650 sh, 1 640 sh, 1 630 s (CO), 1 600 s, 1 570 s $cm^{-1}$. $^1$H-NMR ($CDCl_3$) : δ = 1.26 (t, J = 7 Hz ; 3H, $CH_3$—$CH_2$), 3.03 (t, J = 6,5 Hz ; 2H, Ar—$CH_2$), 3.33-3.67 (m, 3H, $CH_2$—CH), 3.86 (s, 7H, 2 x $OCH_3$ und 1 OH), 4.08 (t, J = 6,5 Hz ; 2H, $CH_2$—$CH_2$—N), 6.87 (s, 1H, 3—H), 7.08 (s, 1H, 7—H), 7.62 (s, 1H, 10—H), 10.17 (s, 1H, CHO), 10.38 (t, J = 5 Hz ; 1H, NHCO).

$C_{19}H_{22}N_2O_5$ (358.40)

Ber.: C 63.67 H 6.18 N 7.82 % ;
Gef.: C 63.59 H 6.11 N 7.81 %.

Beispiel 6

1-Formyl-5,6-dihydro-8,9-dimethoxy-pyrrolo[2,1-a]isochinolin-2-carbonsäure-(2-furfuryl)-amid

durch Umlagerung von 3-[3,4-Dihydro-6,7-dimethoxy-isochinolinyl-(1)]-furan-4-carbonsäure-(2-furfuryl)-amid-hydrochlorid mit Morpholin.

Ausbeute 78 % ; rotorange Kristalle ; Schmelzpunkt 194-195 °C (aus Methylenchlorid/Ether).

IR : 3 600-3 250 m br (NH), 3 080 w, 3 060 m, 1 670 sh, 1 650 s (CO), 1 600 m, 1 560 s $cm^{-1}$. $^1$H-NMR ($CDCl_3$) : δ = 2.89 (t, J = 6,5 Hz ; 2H Ar—$CH_2$), 3.84 (t, J = 6,5 Hz ; 2H, $CH_2$—$CH_2$—N), 3.83, 3.91 jeweils (s, 3H, $OCH_3$), 4.68 (s, 2H, $CH_2$—NH), 6.36, 6.39 und 7.35 jeweils (m, 1-Furan-H), 6.76 (s, 1H, 3—H), 6.86 (s, 1H, 7—H), 7.91 (s, 1H, 10—H), 9.09 (s, 1H, CHO).

$C_{21}H_{20}N_2O_5$ (380.40)

Ber.: C 66.28 H 5.30 N 7.40 % ;
Gef.: C 66.11 H 5.30 N 7.28 %.

Beispiel 7

1-Formyl-5,6-dihydro-8,9-dimethoxy-pyrrolo[2,1-a]isochinolin-2-carbonsäure-(2-methoxyethyl)-amid

durch Umlagerung von 3-[3,4-Dihydro-6,7-dimethoxy-isochinolinyl-(1)]-furan-4-carbonsäure-(2-methoxyethyl)amid-hydrochlorid mit 2-Methoxyethylamin (Reaktionszeit 16 Stunden).

Ausbeute 78 % ; Schmelzpunkt 142-145 °C (Essigester/Petrolether).

IR : 3 240 w, 3 200 m (NH), 3 140 m, 3 075 m, 1 740 m (CO), 1 640 s (CONH), 1 580s, 1 530 s $cm^{-1}$. $^1$N-

NMR ($CDCl_3$) δ = 3.02 (t, $\underline{J}$ = 6,5 Hz ; 2H, Ar—$C\underline{H}_2$), 3.44 (s, 3H, $OC\underline{H}_3$), 3.61, 3.64 jeweils (s, 2H, NH—$C\underline{H}_2$—$C\underline{H}_2$—O), 3.93, 3.96 jeweils (s, 3H, $OC\underline{H}_3$), 4.04 (t, $\underline{J}$ = 6,5 Hz ; 2H, $CH_2$—$C\underline{H}_2$—N), 6.83 (s, 1H, $\underline{3—H}$), 7.07 (s, 1H, $\underline{7—H}$), 7.56 (s, 1H, $\underline{10—H}$), 10.13 (s, 1H, $C\underline{H}O$). MS : m/z = 358 (20,5 % $M^+$), 330 (72 %, M— CO), 300 (9,6 %, M—CH = CH—$OCH_3$), 284 (41 %, M— $NHCH_2CH_2OCH_3$), 256 (22,9 %, 284- CO).

$C_{19}H_{22}N_2O_5$ (358.40)

| | | | |
|---|---|---|---|
| Ber.. | C 63.67 | H 6.19 | N 7.82 % ; |
| Gef. : | C 63.65 | H 6.46 | N 7.72 %. |

## Beispiel 8

1-Formyl-5,6-dihydro-8,9-dimethoxy-pyrrolo[2,1-a]isochinolin-2-carbonsäure-[2-(3,4-dimethoxyphenyl)-ethyl]-amid

durch Umlagerung von 3-[3,4-Dihydro-6,7-dimethoxy-isochinolinyl-(1)]-furan-4-carbonsäure-[2-(3,4-dimethoxyphenyl)-ethyl]-amid-hydrochlorid mit Piperidin.

Ausbeute 1,6 g (68 %) rotorange Kristalle ; Schmelzpunkt 154-155 °C (aus Essigester).

IR : 3 400 br w, 1 740 m (CHO), 1 670 s (NHCO), 1 650 s, 1 570 s $cm^{-1}$. $^1H$-NMR ($CDCl_3$) : δ = 2.71-3.18 (m, 4H, 2 x Ar—$C\underline{H}_2$), 3.78 (s, 9H, 3 x $OC\underline{H}_3$), 3.92 (s, 3H, $OC\underline{H}_3$), 3.58-4.07 (m, 4H, 2 x $CH_2$—$CH_2$—N), 6.60-6.87 (m, gH, 4 Aromaten-H, 1 Pyrrol-H, 1 $N\underline{H}CO$), 7.53 (s, 1H, $\underline{10—H}$), 9.22 (s, 1H, $C\underline{H}O$). MS : m/z = 464 (68 %, $M^+$), 313 (100 %, M— $CH_2C_6H_3(OCH_3)_2$), 285 (36 %, 313- CO).

$C_{26}H_{28}N_2O_6$ (464,54)

| | | | |
|---|---|---|---|
| Ber. : | C 67.22 | H 6.08 | N 6.03 % ; |
| Gef. : | C 67.32 | H 6.31 | N 6.19 %. |

**Patentansprüche**

1. Verfahren zur Herstellung von 5,6-Dihydro-pyrrolo[2,1-a]-isochinolinen der allgemeinen Formel I

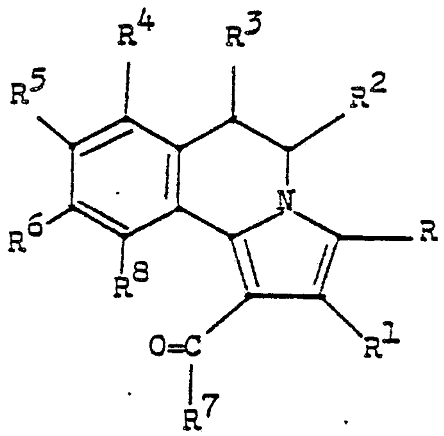

(I)

worin

R, $R^2$, $R^3$, $R^7$, die gleich oder verschieden sein können, Wasserstoff oder Alkyl mit 1 bis 4 C-Atomen,

$R^1$ Cyano, Hydroxycarbonyl, Alkoxycarbonyl mit 1 bis 4 C-Atomen, eine primäre Aminocarbonylgruppe oder eine sekundäre Aminocarbonylgruppe R'—NH—CO—, in der der Substituent R' eine gegebenenfalls substituierte aliphatische, cycloaliphatische araliphatische, aromatische oder heterocyclische Gruppe ist,

$R^4$ und $R^8$, die gleich oder verschieden sein können, Wasserstoff, Hydroxy, Alkoxy, Alkylthio mit jeweils bis zu 4 C-Atomen oder eine —$NR^9R^{10}$-Gruppe,

$R^5$ und $R^6$, die gleich oder verschieden sein können, Hydroxy, Alkoxy oder Alkylthio mit jeweils 1 bis 4 C-Atomen oder gemeinsam eine —O—$CH_2$—O—, —O—$CH_2$—$CH_2$—O— oder —O—CH = CH—O-Gruppe,

$R^9$ Wasserstoff oder Alkyl mit 1 bis 4 C-Atomen und

$R^{10}$ gegebenenfalls durch Hydroxy, Methoxy oder Furfuryl substituiertes Alkyl mit 1 bis 4 C-Atomen bedeuten, dadurch gekennzeichnet, daß man ein 1-(3-Furyl)-3,4-dihydro-isochinolin der allgemeinen Formel II

(Siehe Formel II Seite 7 f.)

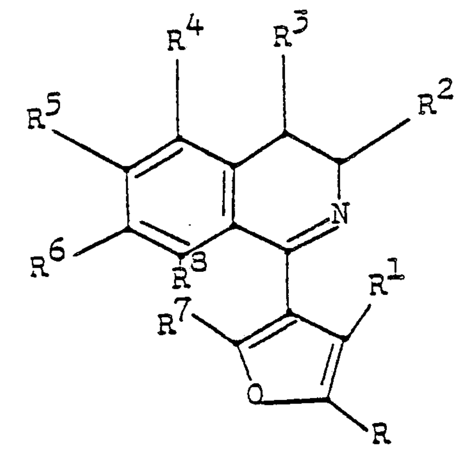

(II)

worin R sowie $R^1$ bis $R^8$ die oben angegebenen Bedeutungen besitzen, in einem primären oder sekundären Amin auf mindestens 80 °C mehrere Stunden erhitzt, das Amin entfernt, das Reaktionsprodukt mit verdünnter Säure behandelt und das Reaktionsprodukt reinigt und isoliert.

2. Verfahren nach Anspruch 1, dadurch gekennzeichnet, daß man als Amin Morpholin, Piperidin, 2-(3,4-Dimethoxyphenyl)-ethylamin, 1-Amino-2-propanol oder Methoxy-ethylamin verwendet.

3. Verfahren nach Anspruch 1 oder 2, dadurch gekennzeichnet, daß man das Reaktionsprodukt mit verdünnten Mineralsäuren behandelt.

4. Verfahren nach einem der Ansprüche 1 bis 3, dadurch gekennzeichnet, daß man von einer Verbindung der allgemeinen Formel II ausgeht, in der

R, $R^2$, $R^3$, $R^4$, $R^7$, $R^8$ jeweils Wasserstoff,

$R^1$ Cyano, Hydroxycarbonyl, Alkoxycarbonyl mit 1 bis 4 C-Atomen oder substituiertes Niederalkylaminocarbonyl und

$R^5$ und $R^6$, die gleich oder verschieden sein können, Hydroxy, Alkoxy mit 1 bis 4 C-Atomen, oder gemeinsam eine $-O-CH_2-O-$, $-O-CH_2-CH_2-O-$ oder $-O-CH=CH-O$-Gruppe bedeuten.

5. Verfahren nach einem der Ansprüche 1 bis 3, dadurch gekennzeichnet, daß man von einer Verbindung der allgemeinen Formel II ausgeht, in der

R, $R^2$, $R^3$, $R^4$, $R^7$, $R^8$ jeweils Wasserstoff,

$R^1$ Cyano, Hydroxycarbonyl, Methoxycarbonyl,

Ethoxycarbonyl, $-CO-NHCH_2CH(OH)CH_3$, 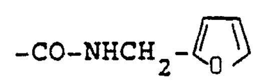 ,

$-CO-NHCH_2CH_2OCH_3$, 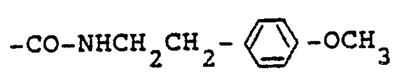 oder

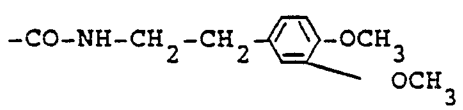 ,

$R^5$ Methoxy und

$R^6$ Wasserstoff, Methoxy oder gemeinsam mit $R^5$ eine $-O-CH_2-O$-Gruppe bedeuten.

**Claims**

1. Process for preparing 5,6-dihydro-pyrrolo[2,1-a]-isoquinolines of general formula I

(See formula I page 8)

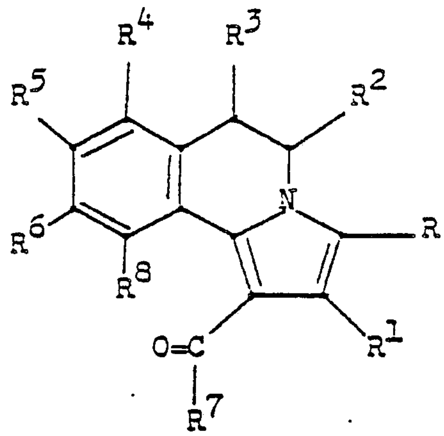

(I)

wherein

R, $R^2$, $R^3$ and $R^7$, which may be identical or different, represent hydrogen or alkyl with 1 to 4 carbon atoms,

$R^1$ represents cyano, hydroxycarbonyl alkoxycarbonyl with 1 to 4 carbon atoms, a primary aminocarbonyl group or a secondary aminocarbonyl group R'—NH—CO—, wherein the substituent R' represents an optionally substituted aliphatic, cycloaliphatic, araliphatic, aromatic or heterocyclic group,

$R^4$ and $R^8$, which may be identical or different, represent hydrogen, hydroxy, alkoxy, alkylthio with up to 4 carbon atoms or an $—NR^9R^{10}$ group,

$R^5$ and $R^6$, which may be identical or different, represent hydroxy, alkoxy or alkylthio with 1 to 4 carbon atoms or together represent an $—O—CH_2—O—$, $—O—CH_2—CH_2—O—$ or $—O—CH=CH—O—$ group,

$R^9$ represents hydrogen or alkyl with 1 to 4 carbon atoms and

$R^{10}$ represents alkyl with 1 to 4 carbon atoms optionally substituted by hydroxy, methoxy or furfuryl, characterised in that a 1-(3-furyl)-3,4-dihydro-isoquinoline of general formula II

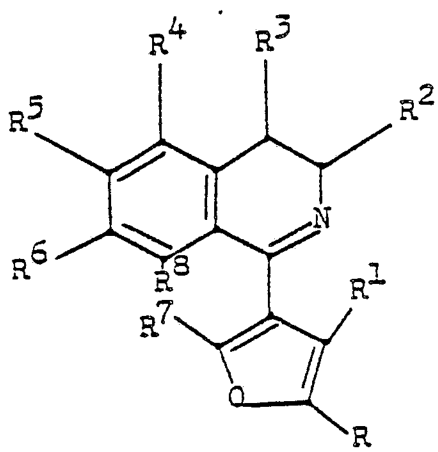

(II)

wherein R and $R^1$ to $R^8$ have the meanings given hereinbefore, is heated to at least 80 °C in a primary or secondary amine for several hours, the amine is removed, the reaction product is treated with dilute acid and the reaction product is purified and isolated.

2. Process as claimed in claim 1, characterised in that morpholine, piperidine, 2-(3,4-dimethoxyphenyl)-ethylamine, 1-amino-2-propanol or methoxy-ethylamine is used as the amine.

3. Process as claimed in claim 1 or 2, characterised in that the reaction product is treated with dilute inorganic acids.

4. Process as claimed in one of claims 1 to 3, characterised in that the starting material used is a compound of general formula II wherein

R, $R^2$, $R^3$, $R^4$, $R^7$ and $R^8$ each represent hydrogen,

$R^1$ represents cyano, hydroxycarbonyl, alkoxycarbonyl with 1 to 4 carbon atoms or substituted lower alkylaminocarbonyl and

$R^5$ and $R^6$, which may be identical or different, represent hydroxy, alkoxy with 1 to 4 carbon atoms, or together represent an $—O—CH_2—O—$, $—O—CH_2—CH_2—O—$ or $—O—CH=CH—O—$ group.

5. Process as claimed in one of claims 1 to 3, characterised in that the starting material used is a compound of general formula II wherein

R, $R^2$, $R^3$, $R^4$, $R^7$ and $R^8$ each represent hydrogen,

$R^1$ represents cyano, hydroxycarbonyl methoxycarbonyl,

Ethoxycarbonyl, $-CO-NHCH_2CH(OH)CH_3$, 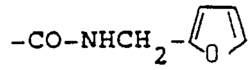,

$-CO-NHCH_2CH_2OCH_3$, 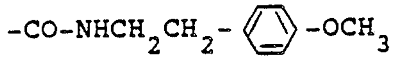 or

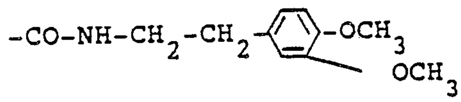,

$R^5$ represents methoxy and
$R^6$ represents hydrogen or methoxy or together with $R^5$ it represents an $-O-CH_2-O-$ group.

## Revendications

1. Procédé pour la préparation de 5,6-dihydro-pyrrolo[2,1-a]-isoquinoléines de formule générale I

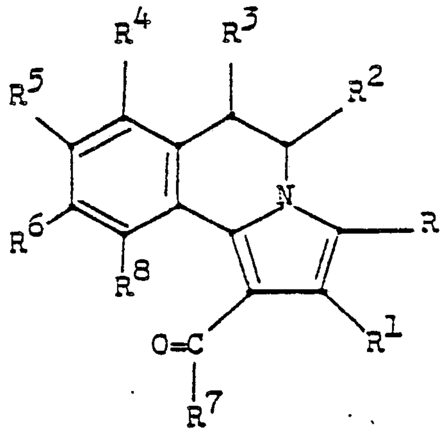

(I)

dans laquelle

R, $R^2$, $R^3$, $R^7$ qui peuvent être identiques ou différents, représentent hydrogène ou alcoyle avec 1 à 4 atomes de C,

$R^1$ représente cyano, hydroxycarbonyle, alcoxycarbonyle avec 1 à 4 atomes de C, un groupe aminocarbonyle primaire ou un groupe aminocarbonyle secondaire R'—NH—CO, dans lequel le substituant R' est un groupe aliphatique, cycloaliphatique, araliphatique, aromatique ou hétérocyclique, éventuellement substitué,

$R^4$ et $R^8$ qui peuvent être identiques ou différents, représentent hydrogène, hydroxy, alcoxy, alcoylthio avec chacun jusqu'à 4 atomes de C ou un groupe $-NR^9R^{10}$,

$R^5$ et $R^6$ qui peuvent être identiques ou différents, représentent hydroxy, alcoxy ou alcoylthio avec chacun 1 à 4 atomes de C ou ensemble un groupe $-O-CH_2-O-$, $-O-CH_2-CH_2-O-$ ou $-O-CH=CH-O-$,

$R^9$ représente hydrogène ou alcoyle avec 1 à 4 atomes de C et

$R^{10}$ représente alcoyle avec 1 à 4 atomes de C, éventuellement substitué par hydroxy, méthoxy ou furfuryle, caractérisé en ce qu'on chauffe une 1-(3-furyl)-3,4-dihydro-isoquinoléine de formule générale II

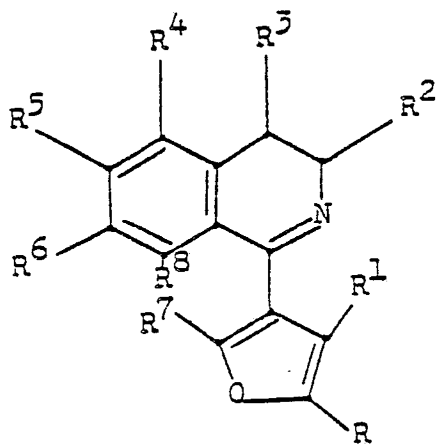

(II)

dans laquelle R ainsi que $R^1$ à $R^8$ possèdent les significations indiquées plus haut, dans une amine primaire ou secondaire à au moins 80 °C, pendant plusieurs heures, en ce qu'on élimine l'amine, traite le produit de la réaction par un acide dilué et purifie le produit de réaction et l'isole.

2. Procédé selon la revendication 1, caractérisé en ce que l'on utilise comme amine la morpholine, la pipéridine, la 2-(3,4-diméthoxyphényl)-éthylamine, le 1-amino-2-propanol ou la méthoxy-éthylamine.

3. Procédé selon la revendication 1 ou 2, caractérisé en ce que l'on traite le produit de la réaction par des acides minéraux dilués.

4. Procédé selon l'une des revendications 1 à 3, caractérisé en ce qu'on part d'un composé de formule générale II, dans laquelle

R, $R^2$, $R^3$, $R^4$, $R^7$, $R^8$ représentent chacun hydrogène,

$R^1$ représente cyano, hydroxycarbonyle, alcoxycarbonyle avec 1 à 4 atomes de C ou alcoyl (inférieur) aminocarbonyle substitué et

$R^5$ et $R^6$ qui peuvent être identiques ou différents, représentent hydroxy, alcoxy avec 1 à 4 atomes de C ou ensemble un groupe $—O—CH_2—O—$, $—O—OH_2—CH_2—O—$ ou $—O—CH = CH—O—$.

5. Procédé selon l'une des revendications 1 à 3, caractérisé en ce qu'on part d'un composé de formule générale II dans laquelle

R, $R^2$, $R^3$, $R^4$, $R^7$, $R^8$ représentent chacun hydrogène,

$R^1$ représente cyano, hydroxycarbonyle méthoxycarbonyle,

Ethoxycarbonyl, $-CO-NHCH_2CH(OH)CH_3$, 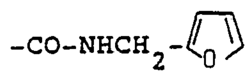 ,

$-CO-NHCH_2CH_2OCH_3$, 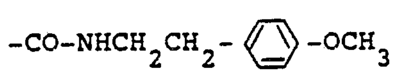 ou

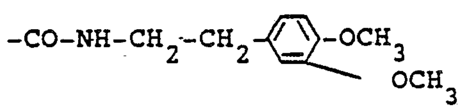 ,

$R^5$ représente méthoxy et

$R^6$ représente hydrogène, méthoxy ou ensemble avec $R^5$ un groupe $—O—CH_2—O—$.